# EUROPEAN PATENT APPLICATION

(11) **EP 2 186 523 A1**
(43) Date of publication of application: **19.05.2010**
(21) Application number: 08450185.7
(22) Date of filing: 18.11.2008
(51) Int. Cl.: A61K 36/49, A61K 36/77, A61K 31/704, A61P 43/00

(54) **Use of escin for treatment of Type IV hypersensitivity reaction**

(71) Applicant: Marinomed Biotechnologie GmbH, 1210 Vienna (AT)
(72) Inventor: Prieschl-Grassauer, Eva, 1220 Vienna (AT)
(74) Representative: Alge, Daniel

(57) **Abstract**

The present invention provides the use of escin for the manufacture of a pharmaceutical preparation for the treatment of a Type IV hypersensitivity reaction or symptoms of a Type IV hypersensitivity reaction, such as diseases selected from contact dermatitis, atopic dermatitis, hypersensitivity pneumonitis, chronic transplant rejection, graft versus host disease, cell mediated autoimmune diseases, Hashimoto's thyroiditis, Sjogren's disease, adrenalitis, polymyositis, or pernicious anemia.

## Description

The present invention relates to the field of immunology.

The present invention is of immunological impact. According to the Coombs and Gell classification system, four types of hypersensitivity reactions can be defined: Type I also referred to as classic immediate allergy reaction is mediated by Immunoglobulin E (IgE) class antibodies. Type II hypersensitivity mode of function is cytotoxic, its mechanisms depend on antibodies of classes IgM or IgG and the Complement system. Type III immune complex diseases also are induced via IgG and the Complement system. In contrast to the mentioned immediate Type reactions hypersensitivities of Type IV are delayed and T cells, not antibodies act as mediators.

Symptoms of Type IV hypersensitivity reactions include mild ones like a runny nose, but also more severe conditions like poison ivy rash, (contact-) dermatitis, hypersensitivity pneumonitis or allograft rejection. Pharmaceutical treatment varies, including over-the-counter or prescription corticosteroid preparations, injectable or oral corticosteroids, and Burrow's solution, a preparation made of aluminum acetate dissolved in water. Clinically administered, corticosteroids suppress the immune system, Burrow's solution has astringent and antibacterial properties. Therapies can cause side effects, notably in case of prolonged usage. Numbers of Type IV hypersensitivities are worldwide on the rise and extremely common.

The first delayed type hypersensitivity reaction described used only the tuberculin antigen (tuberculin reaction), but the definition was later expanded to include cell mediated reactions to other bacterial and viral antigens, responses to pure protein with adjuvant or haptens, and host responses to allografts. This reaction has been shown to be absolutely dependent on the presence of memory T cells. Both the CD4+ and CD8+ fractions of cells have been shown to modulate a response. The term "chronic transplant rejection" is reserved for cases where the rejection is due to a chronic immune response against the transplanted tissue. This often leads to the need of a new organ transplant after approximately 10 years.

Graft versus host disease is a result of cellular immunity and is an example of a delayed type hypersensitivity response. Similar to the graft vs. host disease form of cell mediated immunity are some autoimmune diseases: Hashimoto's thyroiditis, Sjogren's disease, adrenalitis, polymyositis, and pernicious anemia. The pathological picture is one of mononuclear cell infiltration and tissue destruction. Finally, it is necessary to view delayed type hypersensitivity not as an individual phenomenon but rather as a group of related responses to antigen. These include the tuberculin reaction, Jones-Mote reaction, contact hypersensitivity and graft rejection. These reactions can be further divided into CD4+ and CD8+ compartments and then subdivided on the basis of the T cells' cytokine secretion patterns.

Delayed hypersensitivity Type IV reaction is an inflammatory response that develops 24 to 72 hours after exposure to an antigen the immune system recognizes as foreign. The reaction is mediated by T cells rather than by antibodies. Helper T (Th 1) cells produce cytokines like interferon gamma, interleukin (IL)-2, and tumor necrosis factor-beta and promote a cell-mediated immune response.

A goal of the present invention is to provide medications which are effective against hypersensitivity Type IV reactions of the immune system.

The present invention provides the use of escin for the manufacture of a pharmaceutical preparation (or a medicament) for the treatment of Type IV hypersensitivity reaction or symptoms of a Type IV hypersensitivity reaction. Therefore, the present invention relates to the use of escin for the manufacture of a pharmaceutical preparation for the treatment of a disease, wherein the origin of the disease is mediated or caused by a Type IV hypersensitivity reaction.

Escin, further notations escine, aescin or aescine, is a triterpene saponin with anti-inflammatory, vasoconstrictor and vasoprotective effects. Beta-escin is the preferred variant of escin according to the present invention. Escin is the major active compound in extracts of the horse chestnut Aesculus hip-pocastanum, and is responsible for most of its medicinal properties. It is a component of a number of pharmaceutical products, actual examples available in Austria are Reparil® (Madaus, Vienna), Opino® (Dr. Kolassa + Merz, Vienna) and Venosin® (Klinge Pharma, Vienna). Cited primary clinically significant activity is in chronic venous insufficiency (CVI), here it attenuates the inflammation response, the attraction of neutrophils, damage to the veins and the release of growth factors.

Escin has been studied extensively both in preclinical models and in CVI affected patients. An extensive review on the pharmacology, pharmacokinetics and therapeutic profile of escin has been compiled by Sirtori C.R. (Pharmacological Research, Vol. 44 (3) 2001:183-193). According to this source, beta-escin is the active component of the extract responsible for the reduction of vascular permeability which can reduce the density of leucocytes in affected tissue. Pharmaceutical products containing escin are also licensed for the therapy of traumatic injuries, (post-operative) oedema, haematoma, bruises, sprain, tendosynovitis and pain in the context of spine injuries. Furthermore, escin possesses significant anti-inflammatory properties, explained by the interference with the leukocyte recruitment and activation.

The use of escin is covered in a multidisciplinary review by Tiffany N. et al. (Journal of Herbal Pharmacotherapy, Vol. 2 (1) 2002:71-85), a systematic review performed by the Cochrane Collaboration discusses efficacy, effectiveness and safety of horse chestnut extract for CVI indication (Cochrane Database Syst. Rev., Vol. 1 2006:CD003230; PMID: 16437450). The mode of action of escin on a molecular level is still unclear, further research is therefore suggested.

Matsuda H. et al. (Bioorg Med Chem Lett 7 (13) 1997: 1611-1616) mention an anti-inflammatory effect of isolated escin compounds from horse chestnut, which is based on vascular constriction measured through an anti-swelling effect in carragenin induced oedemas in rats.

Dattner A.M. (Dermatologic Therapy 16 (2003):106-113) discusses horse chestnuts in the field of herbal medicine with an anti-inflammatory and vasoprotective effect. An elastase-inhibitory activity is attributed to escin.

WO 2008/015007 describes escin for the treatment of diseases mediated or caused by activated granulocytes, preferably a Type I or Type III hypersensitivity reaction or septic shock.

However, none of these publications could show that escin is suitable for the treatment of Type IV hypersensitivity reactions.

According to other sources escin has proinflammatory effects. For example, it is used as adjuvant in several pharmaceutical preparations (US 7,049,063, US 7,033,827, US 6,943,236, US 6,894,146, US 6,858,204, US 6,800,746, US 6,759,515, US 6,630,305, US 6,509,448, US 6,504,010). An adjuvant is an agent which, while not having any specific antigenic effect in itself, may stimulate the immune system, increasing the response to a vaccine. Thus, escin has in certain conditions also immunostimulatory uses.

The present invention provides the use of escin for the manufacture of a pharmaceutical preparation (or a medication) for the therapy of ailments mediated or caused by Type IV hypersensitivity reactions. According to the invention, escin is also provided for the treatment (or prevention, prophylactic treatment) of diseases mediated or caused by a Type IV hypersensitivity reaction or for the treatment (or prevention) of a disease, wherein the origin of the disease is related to a Type IV hypersensitivity reaction. Preferably, the Type IV hypersensitivity reaction comprises a medical condition for example diseases, disorders or ailments, to be treated in the context of a Type IV hypersensitivity reaction. In particular, the Type IV hypersensitivity reaction is of a Type IV hypersensitivity disease, or symptoms of the Type IV hypersensitivity reaction or disease. The embodiments, however, do not extend to the treatment of granulocyte mediated diseases, in particular type I or III hypersensitivity reactions, inflammation or of oedemas.

In these embodiments "prevention" should not be interpreted as an absolute success in the sense that a patient can never develop an associated disease, reaction or condition but as the reduction of the chance of developing the disease, reaction or condition in a prophylactic treatment. Prevention by prophylactic treatment is to be understood in the sense of a reduction of the risk of development of Type IV hypersensitivity reaction associated diseases not as a total risk avoidance.

In a special aspect chemical properties of the escin formulation contribute to an optimized allocation of the active components and, furthermore, to a protection of affected mucous membranes due to escin's ability to influence surface tension parameters.

Although any form of escin, e.g. escin extracts, can be used it is preferred to include beta-escin or use beta-escin in the pharmaceutical preparation of the invention. Pharmaceutical formulations containing escin as sole active component or in combination with others have been marketed worldwide for many years and have a remarkable good safety profile. The present invention discloses the treatment (or prevention by prophylactic treatment) of a Type IV hypersensitivity reaction, contact dermatitis or chronic transplant rejection with a pharmaceutical preparation comprising escin.

Escin inhibits dose-dependently an LPS mediated release of TNF alpha in primary human blood. It also reduces the effects of strong hypersensitivity reactions in an in vivo model. Surprisingly, it could be shown for the first time that escin reduced induced topical inflammation in a mouse model of contact dermatitis.

Preferably, the Type IV hypersensitivity reaction is a chronic hypersensitivity reaction or chronic disease. Therefore, the use of escin either alone or in combination with other drugs is an attractive option for chronic patients. In a special embodiment the hypersensitivity disease is a delayed Type IV reaction mediated by cells not by antibodies. The Type IV hypersensitivity reaction is in particular mediated or caused by T cells including CD8+ cells and/or CD4+ cells, in particular Th1 and/or Th2 cells.

In further embodiments the formulation is used for treatment of hypersensitivity reactions from any one of contact dermatitis, atopic dermatitis, hypersensitivity pneumonitis, chronic transplant reaction, graft versus host disease, cell mediated autoimmune diseases Hashimoto's thyroiditis, Sjogren's disease, adrenalitis, polymyositis, or pernicious anemia.

Preferably, the pharmaceutical preparation is in the form of a formulation for topical or mucosal application, preferably lotions, cremes, ointments, powders, coverings, patches, band-aids, sprays, dispersion media and gargles. The escin preparation is especially suitable for topical application to treat skin or mucosal symptoms of the hypersensitivity mediated disease. But also systemic treatment, e.g. parenteral or oral (also for specific mucosal treatment), is possible.

A further embodiment is **characterized in that** the preparation is intended for oral intake, preferably in the form of pastilles, tablets, troches, lozenges, pills, gums, powders or drinking solutions. Systemic or topical distribution of escin can be facilitated by formulations and carriers known in the state of the art.

The preparation may also comprise pharmaceutical carriers, excipients, preferably polymeric excipients, or additives. The term "carrier" refers to a diluent, e.g. water, saline, excipient, or vehicle, with which the composition can be administered. For a solid or fluid composition the carriers or additives in the pharmaceutical composition may comprise SiO₂ TiO₂, a binder, such as microcrystalline cellulose, polyvinylpyrrolidone (polyvidone or povidone), gum tragacanth, gelatine, starch, lactose or lactose monohydrate, alginic acid, maize (corn) starch and the like; a lubricant or surfactant, such as magnesium stearate, or sodium lauryl sulphate; a glidant, such as colloidal silicon dioxide; a sweetening agent, such as sucrose or saccharin. Preferably, the preparation comprises buffers or pH adjusting agents, e.g. selected from citric acid, acetic acid, fumaric acid, hydrochloric acid, malic acid, nitric acid, phosphoric acid, propionic acid, sulfuric acid, tartaric acid, or combinations thereof. Escin in the form of a pharmaceutically acceptable salt, for example sodium salt, may also be used. Other pharmaceutically acceptable salts include, among others, potassium, lithium and ammonium salts. Preferred excipients are polymers, especially cellulose and cellulose derivatives.

In a further embodiment the preparation comprises pharmaceutical carriers, excipients, vectors, additives, or adjuvants, preferably of polymeric origin.

Preferably, escin is formulated for administration in doses between 0.01 mg/kg body weight of a patient and 500 mg/kg, preferably between 0.1 mg/kg and 100 mg/kg, most preferred between 1 mg/kg and 40 mg/kg. The present invention also provides for the use of the pharmaceutical preparations. The preparation is not limited for to be administered at the same time of a type IV hypersensitivity reaction occurs but can also be used before or after the reaction, e.g. for prophylactic treatment, i.e. a treatment before an expected exposure to an immune stimulant to reduce the force of the reaction.

The present invention is further illustrated by the following figure and example, without being limited thereto. Figures:

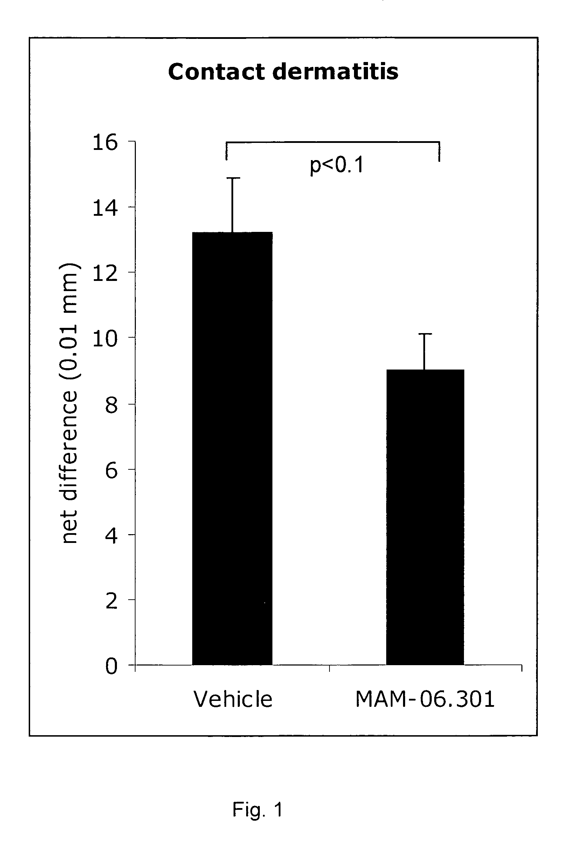

Fig. 1: Efficacy of escin in an oxazalone induced contact dermatitis model. 3 mg/kg of escin were applied intraperitoneally (ip.) 6 h before challenge, 0.9% saline solution served as a vehicle. The y-axis displays the net difference in 0.01 mm between the ear thickness of the challenged ear compared to the vehicle treated ear. Bars represent the mean of five animals, SEM is indicated.

### Example:

Escin was evaluated for anti-inflammatory activity by ip. injection in the oxazolone induced topical inflammation in BALB/c mice, which is a model of type IV hypersensitivity reaction, in particular contact dermatitis. The swelling of the challenged ear in comparison to the unchallenged control was determined as a parameter for induced contact dermatitis. Based on the results escin caused significant inhibition of oxazolone induced ear swelling 6 hours after oxazolone application.

## Claims

1. Use of escin for the manufacture of a pharmaceutical preparation for the treatment of a Type IV hypersensitivity reaction or symptoms of a Type IV hypersensitivity reaction.

2. Use according to claim 1, **characterized in that** escin is beta-escin.

3. Use according to claim 1 or 2, **characterized in that** the Type IV hypersensitivity reaction comprises a medical condition, such as a disease, disorder or ailment, to be treated in the context of a Type IV hypersensitivity reaction.

4. Use according to any one of claims 1 to 3, **characterized in that** the Type IV hypersensitivity reaction is of a Type IV hypersensitivity disease.

5. Use according to any one of claims 1 to 4, **characterized in that** the reaction is associated with the following conditions, or the disease is selected from the following conditions selected from any one of contact dermatitis, atopic dermatitis, hypersensitivity pneumonitis, chronic transplant rejection, graft versus host disease, cell mediated autoimmune diseases, Hashimoto's thyroiditis, Sjogren's disease, adrenalitis, polymyositis, or pernicious anemia.

6. Use according to claims 1 to 5, **characterized in that** the hypersensitivity reaction is delayed and mediated by T cells.

7. Use according to any one of claims 1 to 6, **characterized in that** the preparation is in the form of a formulation for topical or mucosal use, preferably skin lotions, cremes, ointments, powders, patches, sprays, dispersion media and gargle solutions.

8. Use according to any one of claims 1 to 7, **characterized in that** the preparation is intended for oral intake, preferably in the form of pastilles, tablets, gums, troches, lozenges, pills, powders or drinking solutions.

9. Use according to any one of claims 1 to 8, **characterized in that** the preparation comprises pharmaceutical carriers, excipients, vectors, additives, or adjuvants, preferably of polymeric origin.

10. Use according to any one of claims 1 to 9, **characterized in that** escin is formulated for uses in a dose ranging from 0.01 mg/kg to 500 mg/kg body weight, preferably ranging from 0.1 mg/kg to 100 mg/kg body weight, most preferably from 1 mg/kg to 40 mg/kg body weight.
